# EUROPEAN PATENT SPECIFICATION

(11) **EP 2 972 383 B1**
(45) Date of publication and mention of the grant of the patent: **21.08.2019**
(21) Application number: 14713260.9
(22) Date of filing: 26.02.2014
(51) Int. Cl.: G01N 33/68

(54) **SYSTEM AND METHOD FOR DETERMINING RISK OF PRE-ECLAMPSIA BASED ON BIOCHEMICAL MARKER ANALYSIS**
SYSTEM UND VERFAHREN ZUR BESTIMMUNG EINER PRÄEKLAMPSIE AUF BASIS DER ANALYSE BIOCHEMISCHER MARKER
SYSTÈME ET MÉTHODE DE DÉTERMINATION DU RISQUE DE PRÉ-ÉCLAMPSIE D'APRÈS L'ANALYSE DE MARQUEURS BIOCHIMIQUES

(30) Priority: 15.03.2013 US 201313837134
(43) Date of publication of application: 20.01.2016
(73) Proprietor: WALLAC OY, 20101 Turku (FI)
(72) Inventor: HURSKAINEN, Pertti, 20760 Piispanristi (FI); KORPIMÄKI, Teemu, 20100 Turku (FI); KOURU, Heikki, 21260 Raisio (FI); SAIRANEN, Mikko, 21230 Masku (FI)
(74) Representative: Kirkham, Nicholas Andrew
(86) International application number: PCT/IB2014/059279
(87) International publication number: WO 2014/140975

(56) References cited:
- EP-A1- 2 490 027
- EP-A1- 2 490 027
- WO-A1-2009/097584
- WO-A1-2009/097584
- WO-A1-2009/097584
- WO-A1-2009/108073
- WO-A1-2009/108073
- WO-A1-2009/108073
- WO-A1-2010/059952
- WO-A1-2010/059952
- WO-A2-2014/036440
- WO-A2-2014/036440
- WO-A2-2014/036440
- EDI VAISBUCH ET AL: "Retinol binding protein 4 - a novel association with early-onset preeclampsia", JOURNAL OF PERINATAL MEDICINE, vol. 38, no. 2, 1 January 2010 (2010-01-01), XP055118249, ISSN: 0300-5577, DOI: 10.1515/jpm.2009.140
- SHANGGUAN X ET AL: "Alterations in serum adipocyte fatty acid binding protein and retinol binding protein-4 in normal pregnancy and preeclampsia", CLINICA CHIMICA ACTA, ELSEVIER BV, AMSTERDAM, NL, vol. 407, no. 1-2, 3 September 2009 (2009-09-03), pages 58-61, XP026391767, ISSN: 0009-8981 [retrieved on 2009-06-29]
- JISOOK PARK ET AL: "Discovery of the serum biomarker proteins in severe preeclampsia by proteomic analysis", EXPERIMENTAL AND MOLECULAR MEDICINE, vol. 43, no. 7, 1 January 2011 (2011-01-01), page 427, XP055019907, ISSN: 1226-3613, DOI: 10.3858/emm.2011.43.7.047
- EDI VAISBUCH ET AL: "Retinol binding protein 4 - a novel association with early-onset preeclampsia", JOURNAL OF PERINATAL MEDICINE, vol. 38, no. 2, 1 January 2010 (2010-01-01), XP055118249, ISSN: 0300-5577, DOI: 10.1515/jpm.2009.140
- SHANGGUAN X ET AL: "Alterations in serum adipocyte fatty acid binding protein and retinol binding protein-4 in normal pregnancy and preeclampsia", CLINICA CHIMICA ACTA, ELSEVIER BV, AMSTERDAM, NL, vol. 407, no. 1-2, 3 September 2009 (2009-09-03), pages 58-61, XP026391767, ISSN: 0009-8981 [retrieved on 2009-06-29]
- EDI VAISBUCH ET AL: "Retinol binding protein 4 - a novel association with early-onset preeclampsia", JOURNAL OF PERINATAL MEDICINE, vol. 38, no. 2, 1 January 2010 (2010-01-01), XP055118249, ISSN: 0300-5577, DOI: 10.1515/jpm.2009.140
- SHANGGUAN X ET AL: "Alterations in serum adipocyte fatty acid binding protein and retinol binding protein-4 in normal pregnancy and preeclampsia", CLINICA CHIMICA ACTA, ELSEVIER BV, AMSTERDAM, NL, vol. 407, no. 1-2, 3 September 2009 (2009-09-03), pages 58-61, XP026391767, ISSN: 0009-8981 [retrieved on 2009-06-29]
- JISOOK PARK ET AL: "Discovery of the serum biomarker proteins in severe preeclampsia by proteomic analysis", EXPERIMENTAL AND MOLECULAR MEDICINE, vol. 43, no. 7, 1 January 2011 (2011-01-01), page 427, XP055019907, ISSN: 1226-3613, DOI: 10.3858/emm.2011.43.7.047

## Description

### Background

Pre-eclampsia is a major cause of maternal and perinatal mortality and morbidity. Pre-eclampsia is characterized by high blood pressure and elevated levels of protein in the urine of a pregnant individual. However, by the time these symptoms appear, the disorder has already begun to exert deleterious effects on the mother and fetus. If individuals at risk for pre-eclampsia can be identified prior to symptom development, negative outcomes may be prevented or mitigated. There is a need for tests, systems, and methods for predicting the risk of development of pre-eclampsia during pregnancy.

### Summary

The present disclosure is directed to methods, apparatus, medical profiles and kits useful for determining the risk that a pregnant individual has or will develop pre-eclampsia, including one or both of early-onset pre-eclampsia and severe pre-eclampsia. As is described, this risk can be determined based at least in part on the amount of the biochemical marker retinol binding protein 4 (RBP4) in a biological sample taken from the pregnant individual. Additional biochemical markers, biophysical markers, maternal history parameters, maternal demographic parameters, and/or maternal biophysical measurements can also be used when determining the risk of pre-eclampsia, including one or both of early-onset pre-eclampsia and severe pre-eclampsia, according to methods described herein.

In one aspect, the present disclosure relates to a method for predicting risk of pre-eclampsia in a pregnant individual, the method including measuring one or more biochemical markers including an RBP4 biochemical marker in a blood sample obtained from the pregnant individual to determine one or more biomarker levels including an RBP4 biomarker level, identifying, by a processor of a computing device, for each of the one or more measured biochemical markers, a difference between the measured biomarker level and a corresponding predetermined control level, and, responsive to the identifying, determining, by the processor, a prediction corresponding to a relative risk of the pregnant individual having or developing pre-eclampsia.

In some embodiments, measuring the one or more biochemical markers includes measuring one or more of a PlGF biochemical marker, a P-Selectin biochemical marker, a PAPP-A biochemical marker, an AFP biochemical marker, and a sTNFR1 biochemical marker. The prediction may correspond to a relative risk of the pregnant individual having or developing early-onset pre-eclampsia (Pe34). The prediction may correspond to a relative risk of the pregnant individual having or developing at least one of severe pre-eclampsia (PeG) and severe early-onset pre-eclampsia (PeG34). The difference may includesat least one of a threshold value and a percentage difference. The prediction may be based in part upon at least one maternal history factor of the pregnant individual. The at least one maternal history factor may include one of a gestational age, a weight, a BMI, a family history status, an ethnicity, and a smoking status.

In some embodiments, the prediction may be positive based at least in part upon identifying the RBP4 biomarker level reflects a statistically significant increase in comparison to a respective control level. Determining the prediction may include calculating a risk assessment score. The risk assessment score may include a proportional risk value. The risk assessment score may include a numeric risk score assigned on a scale.

In some embodiments, the pregnant individual is within a first trimester stage of pregnancy at time of obtaining the blood sample. The first trimester stage may range from forty-two days from conception to ninety-seven days from conception.

In some embodiments, the blood sample includes one of a plasma sample and a serum sample. Measuring the one or more biochemical markers may include performing a quantitative immunoassay. Measuring the one or more biochemical markers may include determining a concentration of each respective biochemical marker. Measuring the one or more biochemical markers may include determining a quantity of each respective biochemical marker.

In one aspect, the present disclosure relates to a system for predicting risk of pre-eclampsia in a pregnant individual including an in vitro diagnostics kit including testing instruments for testing a blood sample obtained from the pregnant individual for one or more biochemical markers including an RBP4 biochemical marker, and a non-transitory computer-readable medium having instructions stored thereon, where the instructions, when executed by a processor, cause the processor to retrieve one or more biomarker levels, where each biomarker level of the one or more biomarker levels corresponds to a biochemical marker tested for using the in vitro diagnostics kit, and where the retrieved one or more biomarker levels includes an RBP4 biomarker level, and calculate a risk assessment score corresponding to a relative risk of the pregnant individual having or developing pre-eclampsia, where the risk assessment score is based at least in part upon the RBP4 biomarker level.

In some embodiments, measuring the one or more biochemical markers includes measuring one or more of a PlGF biochemical marker, a P-Selectin biochemical marker, a PAPP-A biochemical marker, an AFP biochemical marker, and a sTNFR1 biochemical marker. The risk assessment score may be based at least in part upon a comparison of the RBP4 biomarker level and a corresponding predetermined control level.

In some embodiments, the instructions cause the processor to, prior to calculating the risk assessment score, access at least one maternal history factor of the pregnant individual. Accessing the at least one maternal history factor of the pregnant individual may include causing presentation of a graphical user interface at a display device, where the graphical user interface includes one or more input fields for submitting maternal history factor information regarding the pregnant individual. Accessing the at least one maternal history factor of the pregnant individual may include importing, from an electronic medical record, the at least one maternal history factor.

In some embodiments, the instructions cause the processor to, after calculating the risk assessment score, cause presentation of the risk assessment score at a display device. Causing presentation of the risk assessment score may include causing presentation of risk assessment information.

In some embodiments, the testing instruments include an assay buffer. The testing instruments may include one or more of a coated plate, a tracer, and calibrators.

In one aspect, the present disclosure relates to a method for predicting risk of pre-eclampsia in a pregnant individual, the method including measuring one or more biochemical markers in a blood sample obtained from the pregnant individual to determine one or more biomarker levels, where a first biomarker of the one or more biochemical markers includes RBP4, and a first biomarker level includes an RBP4 biomarker level, and calculating, by the processor, a risk assessment score corresponding to a relative risk of the pregnant individual having or developing pre-eclampsia, where the risk assessment score is based at least in part upon the RBP4 biomarker level.

In some embodiments, the risk assessment score is based at least in part upon a comparison of the RBP4 biomarker level and a corresponding predetermined control level. Measuring the one or more biochemical markers may include measuring one or more of a PlGF biochemical marker, a P-Selectin biochemical marker, a PAPP-A biochemical marker, an AFP biochemical marker, and a sTNFR1 biochemical marker. Measuring the one or more biochemical markers may include applying mass spectrometry analysis.

In some embodiments, calculating the risk assessment score includes normalizing the comparison of the biomarker level and the corresponding predetermined control level based upon one or more maternal demographic values. Normalizing the comparison may include applying a multiple of mean statistical analysis. Calculating the risk assessment score may include normalizing the comparison of the biomarker level and the corresponding predetermined control level based upon one or more maternal biophysical attributes.

In one aspect, the present disclosure relates to a non-transitory computer readable medium having instructions stored thereon, where the instructions, when executed by a processor, cause the processor to access one or more measurements of one or more biochemical markers, where the measurements were obtained by testing biochemical marker levels in a blood sample obtained from a pregnant individual, a first biomarker of the one or more biochemical markers includes an RBP4 biomarker, and a first measurement of the one or more measurements includes an RBP4 level. The instructions may cause the processor to calculate a risk assessment score corresponding to a relative risk of the pregnant individual having or developing pre-eclampsia, where the risk assessment score is based at least in part upon the RBP4 biomarker level.

In some embodiments, a second biomarker of the one or more biochemical markers is one of a PlGF biochemical marker, a P-Selectin biochemical marker, a PAPP-A biochemical marker, an AFP biochemical marker, and a sTNFR1 biochemical marker. The risk assessment score may be based at least in part on a comparison of the RBP4 biomarker level and a corresponding predetermined control level.

In one aspect the present disclosure relates to a system for predicting risk of pre-eclampsia in a pregnant individual including an in vitro diagnostics kit including testing instruments for testing a blood sample obtained from the pregnant individual for one or more biochemical markers, where a first biomarker of the two or more biochemical markers includes RBP4. The system may include a non-transitory computer-readable medium having instructions stored thereon, where the instructions, when executed by a processor, cause the processor to retrieve one or more biomarker levels, where each biomarker level of the one or more biomarker levels corresponds to a biochemical marker tested for using the in vitro diagnostics kit, and where the retrieved one or more biomarker levels includes an RBP4 biomarker level, and identify, for each of the one or more measured biochemical markers, a difference between the measured biomarker level and a corresponding predetermined control level. The instructions may cause the processor to, responsive to the identifying, determine a prediction corresponding to a relative risk of the pregnant individual having or developing pre-eclampsia.

In some implementations, a second biomarker of the one or more biochemical markers is one of a PlGF biochemical marker, a P-Selectin biochemical marker, a PAPP-A biochemical marker, an AFP biochemical marker, and a sTNFR1 biochemical marker.

In one aspect, the present disclosure relates to a non-transitory computer readable medium having instructions stored thereon, where the instructions, when executed by a processor, cause the processor to access measurements of one or more biochemical markers, where the measurements were obtained by testing biochemical marker levels in a blood sample obtained from a pregnant individual, and a first biomarker of the one or more biochemical markers includes RBP4. The instructions may cause the processor to identify, for each of the one or more measured biochemical markers, a difference between the measured biomarker level and a corresponding predetermined control level, and responsive to the identifying, determine a prediction corresponding to a relative risk of the pregnant individual having or developing pre-eclampsia.

In some embodiments, a second biomarker of the one or more biochemical markers is one of a PlGF biochemical marker, a P-Selectin biochemical marker, a PAPP-A biochemical marker, an AFP biochemical marker, and a sTNFR1 biochemical marker.

### Brief Description of the Figures

The foregoing and other objects, aspects, features, and advantages of the present disclosure will become more apparent and better understood by referring to the following description taken in conjunction with the accompanying drawings, in which:
FIGS. 1A through 1C illustrate box-whisker plots of biochemical marker multiple of the median (MoM) in four pregnancy outcome groups: control, early onset pre-eclampsia, severe pre-eclampsia, and severe early-onset pre-eclampsia;
FIG. 2 is a Receiver Operation Characteristic (ROC) curve for the prediction of pre-eclampsia using the RBP4 biomarker;
FIG. 3 is a table identifying Mahalanobis distances between the control group and case groups;
FIG. 4 is a table identifying detection rates for combinations of biochemical markers in identifying individuals who have or will develop one or both of early onset pre-eclampsia and severe pre-eclampsia;
FIG. 5 is a flow chart of an example method for determining a prediction corresponding to a relative risk of a pregnant individual having or developing one or both of severe pre-eclampsia and early onset pre-eclampsia; and
FIG. 6 is a block diagram of a computing device and a mobile computing device.

The features and advantages of the present disclosure will become more apparent from the detailed description set forth below when taken in conjunction with the drawings, in which like reference characters identify corresponding elements throughout. In the drawings, like reference numbers generally indicate identical, functionally similar, and/or structurally similar elements.

### Detailed Description

In some implementations, the present disclosure may be directed to methods, apparatus, medical profiles and kits useful for determining the risk that a pregnant individual has or will develop pre-eclampsia, including one or both of early-onset pre-eclampsia and severe pre-eclampsia. As is described, this risk can be determined based at least in part on the amount of the biochemical marker retinol binding protein 4 (RBP4) in a biological sample taken from the pregnant individual. Additional biochemical markers, biophysical markers, maternal history parameters, maternal demographic parameters, and/or maternal biophysical measurements can also be used when determining the risk of pre-eclampsia, including one or both of early-onset pre-eclampsia and severe pre-eclampsia, according to methods described herein.

As is described in Example 1, statistical analysis of a clinical population was performed, revealing biochemical marker RBP4 were remarkably effective for determining risk of pre-eclampsia, including one or both of early-onset pre-eclampsia and severe pre-eclampsia, with clinically acceptable detection and false positive rates. As used herein the "% detection" is the percentage-expressed proportion of affected (for example, pre-eclampsia-positive) individuals with a positive result. The "% false positive" is the percentage-expressed proportion of unaffected individuals with a positive result. The predictive power of a marker or combination thereof is commonly expressed in terms of the detection rate for a given false positive rate.

To improve risk evaluation, in some implementations, a number of risk-related factors may be considered in combination with the evaluation of biochemical marker level of an individual. For example, an algorithm for predicting risk of having or developing pre-eclampsia, including one or both of early-onset pre-eclampsia and severe pre-eclampsia, may involve one or more of additional biochemical markers, patient history parameters, patient demographic parameters, and/or patient biophysical measurements. Patient history parameters, in some examples, can include parity, multiple pregnancy, smoking history, past medical conditions, and family history of gestational and/or Type 2 diabetes. Patient demographic parameters, in some examples, can include age, ethnicity, current medications, and vegetarianism. Patient biophysical measurements, in some examples, may include weight, body mass index (BMI), blood pressure, heart rate, cholesterol levels, triglyceride levels, medical conditions (e.g., metabolic syndrome, insulin resistance, atherosclerosis, kidney disease, heart disease, lupus, rheumatoid arthritis, hyperglycemia, dyslipidemia), and gestational age.

In some implementations, to improve evaluation of risk of pre-eclampsia, including one or both of early-onset pre-eclampsia and severe pre-eclampsia, the risk evaluation can be determined based further in part on the amount of one or more of the biochemical markers placental growth factor(PlGF), a P-selectin (e.g., P-selectin, soluble P-selectin (sP-selectin)), pregnancy-associated plasma protein A, pappalysin 1 (PAPP-A), alpha-fetal protein (AFP), and soluble tumor necrosis factor receptor 1 (sTNFR1) in the biological sample taken from the pregnant individual. The selection of a particular combination of additional biochemical markers (e.g., selected from PlGF, sP-selectin, P-selectin, PAPP-A, AFP, and sTNFR1) to be used in a clinical or other laboratory settings can depend on a variety of practical considerations, including the available medical equipment and biochemical marker testing reagents in the particular setting.

As used herein, the term "pre-eclampsia" refers to a condition in a pregnant individual characterized by high blood pressure and protein in the urine. The term "early-onset pre-eclampsia" refers to a pre-eclampsia condition resulting in delivery before gestational week 34. The term "severe pre-eclampsia" refers to a pre-eclampsia condition based on symptoms/diagnostic criteria, including, for example, hypertension, proteinuria, elevated liver enzymes, elevated serum creatinine, low platelet count, sudden weight gain, edema, headache, dizziness, impaired vision, light sensitivity, hyperreflexia, abdominal pain, decreased urine output, nausea, and vomiting. The term "severe early-onset pre-eclampsia" refers to a pregnant individual whose condition fulfills both the characterization of "early-onset pre-eclampsia" and the characterization of "severe pre-eclampsia".

In instances where a pregnant individual is determined to have an increased risk of developing pre-eclampsia, including one or both of early-onset pre-eclampsia and severe pre-eclampsia, using a method as described herein the individual can receive therapy or lifestyle advice from a health care provider. For example, a health care provider may prescribe medication including one or more of a an antihypertensive (e.g., methyldopa, labetalol, a calcium channel blocker), a corticosteroid (e.g., betamethasone, dexamethasone), an antiplatelet drug (e.g., aspirin) or an anticonvulsive (e.g., magnesium sulfate, hydralazine).Additionally, or alternatively, a health care provider may recommend a change in diet, level of physical activity, or bed rest.

Example 1 describes that risk of pre-eclampsia, including one or both of early-onset pre-eclampsia and severe pre-eclampsia ,can be determined using the biochemical marker RBP4, using blood samples that were collected within the first trimester of pregnancy (e.g., up to 14 weeks of gestation). Thus, for use in the methods for detecting pre-eclampsia, including one or both of early-onset pre-eclampsia and severe pre-eclampsia, a sample can be collected between about 9 and 37 weeks gestation, inclusive, including between about 9 and 14 weeks, inclusive, and more generally, prior to about 14 weeks, within first trimester after about 9 weeks, within second trimester and within third trimester. Although earlier testing is often a beneficial policy from a public health perspective, it is understood that collection of samples can sometimes be affected by practical considerations such as a woman delaying a visit to her health care provider until relatively later weeks of gestation.

In certain circumstances, biological samples can be collected on more than one occasion from a pregnant individual, for example, when her risk assessment score requires monitoring for development of pre-eclampsia, including one or both of early-onset pre-eclampsia and severe pre-eclampsia, due to a priori risk, presentation of symptoms and/or other factors. If desired, testing of biochemical markers can be carried out in a home setting, such as by using dipstick biochemical test formats for home use and a personal computing device for interpreting the results.

The methods for determining the risk of pre-eclampsia, including one or both of early-onset pre-eclampsia and severe pre-eclampsia, in a pregnant individual involve determining the amount of the biochemical marker RBP4.

The methods for determining the risk of pre-eclampsia, including one or both of early-onset pre-eclampsia and severe pre-eclampsia, in a pregnant individual involve using a biological sample from the pregnant individual. The biological sample can be any body fluid or tissue sample that contains the selected biochemical marker(s). Example 1 describes use of maternal blood in the form of serum. The choice of biological sample can often depend on the assay formats available in a particular clinical laboratory for testing amounts of the biochemical marker(s). For example, some assay formats lack sensitivity needed for assaying whole blood, such that a clinical laboratory opts for testing a fraction of blood, such as serum, or using dried blood. Exemplary biological samples useful for the methods described herein include blood, purified blood products (such as serum, plasma, etc.), urine, amniotic fluid, a chorionic villus biopsy, a placental biopsy and cervicovaginal fluid. Amounts of the biochemical marker(s) present in a biological sample can be determined using any assay format suitable for measuring proteins in biological samples. A common assay format for this purpose is the immunoassay, including, for example, enzyme immunoassays (EIA) such as enzyme multiplied immunoassay technique (EMIT), enzyme-linked immunosorbent assay (ELISA), IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); dissociation-enhanced lanthanide fluorescent immunoassay (DELFIA) and chemiluminescence assays (CL). Amounts of the biochemical marker(s) present in a biological sample may also be measured by mass spectrometry, for example, by relative or absolute quantitative mass spectrometry using labeled or unlabeled proteins.

To determine whether the amount of biochemical marker(s) is greater than or less than normal, the normal amount of biochemical marker present in a maternal biological sample from a relevant population is determined. The relevant population can be defined based on any characteristics than can affect normal (unaffected) amounts of the biochemical markers. For determining risk of pre-eclampsia, including one or both of early-onset pre-eclampsia and severe pre-eclampsia, the relevant population can be established on the basis of low risk for pre-eclampsia. Once the normal biochemical marker amounts are known, the determined biochemical marker amounts can be compared and the significance of the difference determined using standard statistical methods. When there is a statistically significant difference between the determined biochemical marker amount and the normal biochemical marker amount, there is a significant risk that the tested individual will develop pre-eclampsia, including one or both of early-onset pre-eclampsia and severe pre-eclampsia.

The risk that a pregnant individual develops pre-eclampsia, including one or both of early-onset pre-eclampsia and severe pre-eclampsia, can be determined from biochemical marker amounts using statistical analysis based on clinical data collected in a patient population study. Example 1 shows results from such a study. There are multiple statistical methods for combining parameters that characterize the pregnant individual, such as amounts of the biochemical marker(s), to obtain a risk estimate. The likelihood method (Palomaki and Haddow, 1987) and the linear discriminant function method (Norgarrd-Pedersen et al. Clin. Genet. 37, 35-43 (1990)) are commonly used for this purpose. The basic principle of the likelihood method is that the population distributions for a parameter (such as the amount of a biochemical marker) are known for the 'unaffected' and 'affected' groups. Thus, for any given parameter (such as amount of marker), the likelihood of membership of the 'unaffected' and 'affected' groups can be calculated. The likelihood is calculated as the Gaussian height for the parameter based on the population mean and standard deviation. The 'likelihood ratio' is the ratio of the heights calculated using 'unaffected' and 'affected' population parameters, and is an expression of the increased risk of having a disorder, with respect to a prior risk.

An overview for determining risk in accordance with the methods described herein follows. In current chromosomal abnormality screening practice, biochemical marker values are being referred to smoothed median values to produce adjusted multiple of the median (MoM) values to standardize for factors such as assay, gestation, maternal weight, parity, smoking status, and the like. This is done, for example, because the amounts of biochemical markers in the individual's body change with gestation, in order to calculate risks, the biochemical marker value is adjusted to be unaffected by gestational age. The value of a MoM for a sample is the ratio of the biochemical marker value to the population median value at the same gestational age (or other parameter). The Gaussian heights for biochemical marker results are determined for the 'unaffected' and 'affected' population parameters. The ratio of the height on the 'unaffected' curve and the height on the 'affected' curve is determined. The prior odds are multiplied by this ratio.

In some implementations, to improve identification of risk of a pregnant individual having or developing pre-eclampsia, including one or both of early-onset pre-eclampsia and severe pre-eclampsia, a biological sample is tested for at least one other biochemical marker (e.g., selected from PlGF, a P-selectin, PAPP-A, AFP, and sTNFR1) in addition to RBP4. Conceptually, calculating risk using two or more biochemical markers requires first that individual likelihood ratios be defined for each of the biochemical markers (first corrected for one or more factors such as one or more biophysical markers, maternal history parameters, maternal demographic parameters, and/or maternal biophysical measurements) and then combined (e.g., multiplied) together. In some implementations, an additional factor is introduced in the calculation to account for the extent of overlap of information (correlation) of the two or more individual biochemical markers. For example, r-values may be used to express the correlation between parameters, such as our example of two individual biochemical markers.

As is described in Example 1, statistical analyses of clinical data, including amounts of biochemical marker RBP4, were carried out to determine the risk of a pregnant individual developing pre-eclampsia, including one or both of early-onset pre-eclampsia and severe pre-eclampsia. According to Example 1, for the biochemical marker RBP4, a MoM is calculated in reference to each of early-onset pre-eclampsia, severe pre-eclampsia and severe early-onset pre-eclampsia. The MoM was then adjusted based on parameters including gestational age, patient weight, and cigarette smoking status of each sample.

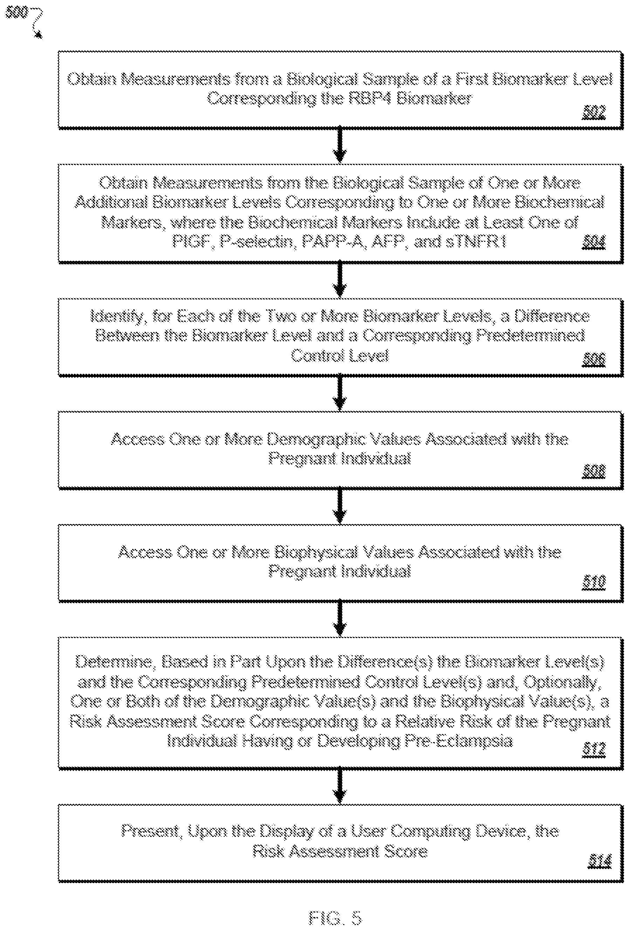

Turning to FIG. 5, a flow chart illustrates an example method 500 for using biomarker level measurements in determining a risk prediction for pre-eclampsia, including one or both of early-onset pre-eclampsia and severe pre-eclampsia, in a pregnant individual. The method 500, for example, may be provided as a software algorithm for use with pre-eclampsia biochemical marker testing (e.g., packaged and/or bundled with a pre-eclampsia diagnostic test kit).

In some implementations, the method 500 begins with obtaining measurements, from a biological sample, of one or more biomarker levels corresponding to a biochemical marker RBP4 (502). The measurements may be obtained in relation to the methods described above for measuring level of RBP4 in a blood sample, such as a plasma sample or a serum sample. The blood sample, for example, may be collected during a first trimester of pregnancy. In some implementations, a clinician or other medical professional enters the measurements into a graphical user interface dialogue of a software application for identifying a risk of a pregnant individual having or developing pre-eclampsia, including one or both of early-onset pre-eclampsia and severe pre-eclampsia. The graphical user interface dialogue, for example, may include one or more drop-down menus, data entry boxes, radio buttons, check boxes, and the like for entering measurements related to the biomarker level as well as, in some embodiments, information regarding the pregnant individual.

In some implementations, for each biomarker of one or more additional biomarkers including at least one of PlGF, a p-Selectin, PAPP-A, AFP, and sTNFR1, measurements of corresponding biomarker level(s) are obtained from the biological sample (504). As described above in relation to obtaining a RBP4 biomarker level, measurements may be obtained in relation to the methods described above for measuring levels of biochemical markers in a blood sample, such as a plasma sample or a serum sample. The blood sample, for example, may be collected during a first trimester of pregnancy. In some implementations, a clinician or other medical professional enters the measurements into a graphical user interface dialogue of a software application for identifying a risk of a pregnant individual having or developing pre-eclampsia, including one or both of early-onset pre-eclampsia and severe pre-eclampsia. The graphical user interface dialogue, for example, may include one or more drop-down menus, data entry boxes, radio buttons, check boxes, and the like for entering measurements related to the biomarker levels as well as, in some embodiments, information regarding the pregnant individual.

In some implementations, for each of the one or more biomarker levels, a difference between the biomarker level and a corresponding predetermined control level is identified (506). The difference, in some examples, can include a threshold difference or a percentage difference between the measurement value and the control value. The predetermined control level, in some implementations, depends at least in part upon profile data obtained in relation to the pregnant individual, such as one or more demographic values and/or one or more biophysical values. In a particular example, the predetermined control level is identified based at least in part upon one or more of an age, a weight (BMI), an ethnicity, and a cigarette smoking status of the pregnant individual. The predetermined control level, in another example, is identified based at least in part upon a gestational age of the pregnant individual's fetus.

In some implementations, one or more demographic values associated with the pregnant individual are accessed (508). In some examples, the demographic values can include one or more of age, ethnicity, current medications, and vegetarianism. The demographic values, in some implementations, may additionally include patient history parameters such as, in some examples, smoking history, past medical conditions, and family history of pregnancy-related disorders, such as pre-eclampsia and gestational diabetes. The demographic values, in some implementations, are accessed via a dialogue interface. For example, a graphical user interface may be presented to a doctor or clinician for entering one or more demographic values related to the pregnant individual. In some implementations, the demographic values are accessed via a medical record system. For example, the demographic values may be imported into the software from a separate (e.g., medical facility) computing system.

In some implementations, one or more biophysical values associated with the pregnant individual are accessed (510). Patient biophysical measurements, in some examples, may include weight, body mass index (BMI), medical conditions, and gestational age. The patient biophysical values, in some implementations, are accessed via a dialogue interface. For example, a graphical user interface may be presented to a doctor or clinician for entering one or more biophysical values related to the pregnant individual. In some implementations, the patient biophysical values are accessed via a medical record system. For example, the patient biophysical values may be imported into the software from a separate (e.g., medical facility) computing system.

In some implementations, a risk assessment score corresponding to a relative risk of the pregnant individual having or developing pre-eclampsia, including one or both of early-onset pre-eclampsia and severe pre-eclampsia, is determined (512). The risk assessment score is based in part upon the biomarker level(s) (e.g., the actual levels and/or a difference between the levels and predetermined control levels). In some implementations, the risk assessment score is based in part upon additional factors, such as the demographic values and/or the biophysical values. The risk assessment score, in some implementations, includes a numeric value corresponding to a proportional risk of the pregnant individual having or developing pre-eclampsia, including one or both of early-onset pre-eclampsia and severe pre-eclampsia. In some implementations, the risk assessment score includes a ranking on a scale (e.g., 1 to 10, 1 to 100, etc.) of a relative risk of the pregnant individual having or developing pre-eclampsia, including one or both of early-onset pre-eclampsia and severe pre-eclampsia. The risk assessment score, in some implementations, includes a percentage likelihood of the pregnant individual having or developing pre-eclampsia, including one or both of early-onset pre-eclampsia and severe pre-eclampsia.

In some implementations, the risk assessment score is presented upon the display of a user computing device (514). The risk assessment score, in some implementations, is presented on a display of a computing device executing the software application for determining risk of pre-eclampsia, including one or both of early-onset pre-eclampsia and severe pre-eclampsia, in a pregnant individual. In some implementations, the risk assessment score is presented as a read-out on a display portion of a specialty computing device (e.g., a test kit analysis device). The risk assessment score may be presented as a numeric value, bar graph, pie graph, or other illustration expressing a relative risk of the pregnant individual having or developing pre-eclampsia, including one or both of early-onset pre-eclampsia and severe pre-eclampsia.

In some implementations, more or fewer steps are included in the method 500, or one or more of the steps of the method 500 may be performed in a different order. For example, in some implementations, demographic values (508) and/or biophysical values (510) are not accessed. In some implementations, rather than identifying a difference between the biomarker level and a corresponding predetermined control level (506), the biomarker level(s) obtained in step(s) 502 (and, optionally, 504) are combined with one or both of demographic value(s) and biophysical value(s) to determine a risk assessment score (512). In other implementations, a difference between the biomarker level and the corresponding predetermined control level (506) is used to determine a prediction (not illustrated) of risk of having or developing pre-eclampsia, including one or both of early-onset pre-eclampsia and severe pre-eclampsia, without generating a risk score in relation to the additional profile values listed in steps 508 and 510. Rather than presenting the risk assessment score on a display of a computing device, in some implementations, a graphic (e.g., "+" for positive, "-" for negative, etc.), a color coding (e.g., red for positive, yellow for indeterminate, green for negative, etc.), or a verbal indication (e.g., as issued via a speaker device in communication with a processor) may be provided as outcome of the analysis. Other modifications of the method 500 are possible.

It is understood that the number values can be different for different study populations, although those shown below provide an acceptable starting point for risk calculations. For example, it has been observed that for a particular clinical center carrying out patient risk analysis, the number values in a risk algorithm can drift over time, as the population in the served region varies over time.

The present disclosure also provides commercial packages, or kits, for determining the risk that a pregnant individual will develop pre-eclampsia, including one or both of early-onset pre-eclampsia and severe pre-eclampsia. Such kits can include one or more reagents for detecting the amount of at least one biochemical marker in a biological sample from a pregnant individual, wherein the at least one biochemical markers include RBP4 as well as, in some implementations, one or more of P1GF, P-selectin, PAPP-A, AFP, and sTNFR1. The diagnostic kit, in some examples, may include one or more of an assay buffer, a coated plate, a tracer, calibrators, instructions for carrying out the test, and software for analyzing biomarker level measurement results in relation to a particular pregnant individual.

### Example 1: Case-Control Study Using Retinol Binding Protein 4 (RBP4) Biochemical Marker for Determining Risk of Pre-Eclampsia in a Pregnant Individual

This example shows use of the RBP4 biochemical marker for determining risk of risk of pre-eclampsia, including one or both of early-onset pre-eclampsia and severe pre-eclampsia, in a pregnant individual.

A retrospective case-control study was undertaken using leftover first trimester maternal serum samples. The dataset included 1000 control samples and 149 cases of pre-eclampsia outcome, including 59 early-onset pre-eclampsia (Pe34), and 90 severe pre-eclampsia (PeG), 50 of which were categorized as severe early onset pre-eclampsia (PeG34). The RBP4 biochemical marker was measured from these samples using an immunoassay.

For the analyses described herein, the measurement results were converted to multiples of median (MoM) by taking into account the gestational age, maternal weight, and cigarette smoking status of the pregnant individual associated with each serum sample.

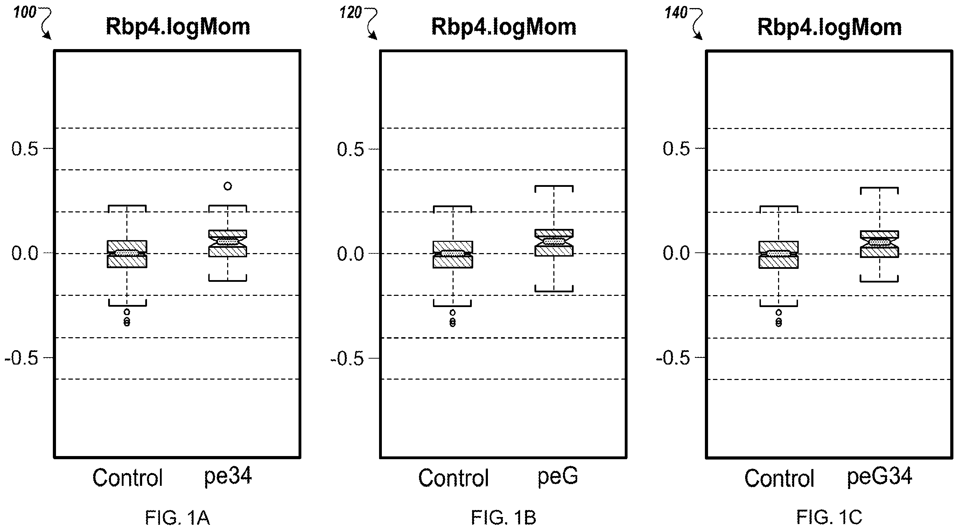

As illustrated in FIG. 1A, a first box-whisker plot 100 of RBP4 multiple of the median (MoM) in a control pregnancy outcome group and an early onset pre-eclampsia outcome group illustrates that the amount of RBP4 in biological samples from pregnant individuals is higher when the individual has an early onset pre-eclampsia outcome in pregnancy. A second box-whisker plot 120 of RBP4 multiple of the median (MoM) in a control pregnancy outcome group and a severe pre-eclampsia outcome group in FIG. 1B illustrates that the amount of RBP4 in biological samples from pregnant individuals is higher when the individual has a severe pre-eclampsia outcome in pregnancy. In reviewing the overlap of both severe and early-onset pre-eclampsia, a third box-whisker plot 120 of FIG. 1C, comparing multiple of the median (MoM) in a control pregnancy outcome group and a severe early-onset pre-eclampsia outcome group, illustrates that the amount of RBP4 in biological samples from pregnant individuals is higher when the individual has a severe early-onset pre-eclampsia outcome in pregnancy. A Mahalanobis distance between the control group and the PeG34 group is about 0.6.

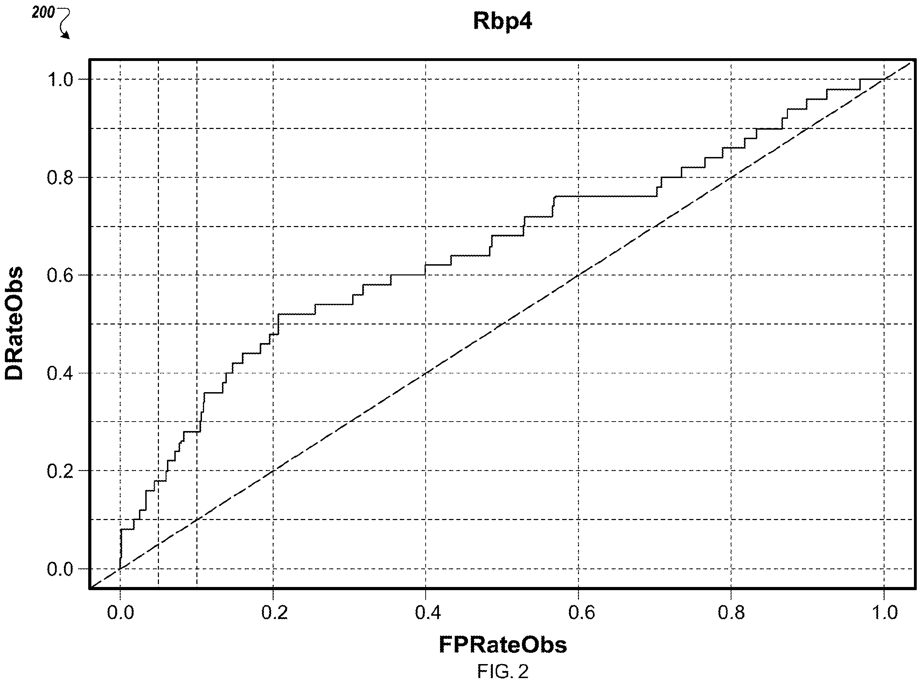

Receiver Operation Characteristic (ROC) analysis of the results of the case study, illustrated in relation to a curve 200 of FIG. 2 demonstrates performance of prediction of severe early-onset pre- using the RBP4 biomarker. Table 1 illustrates data obtained from the curve 200 as well as from similar curves generated in relation to early-onset pre-eclampsia and severe pre-eclampsia. As presented below in relation to Table 1, in screening for one or both of early-onset pre-eclampsia (Pe34) and severe pre-eclampsia (PeG), there was significant independent contributions from maternal blood RBP4. Screening by RBP4 alone, for example, was estimated to identify about 15.3% of individuals developing early-onset pre-eclampsia at a false positive rate of about 5%, increasing to 23.7% identification at a false positive rate of 10% and 35.6% identification at a false positive rate of 15%. In another example, screening by RBP4 was estimated to identify about 17.8% of individuals developing severe pre-eclampsia (PeG) at a false positive rate of about 5%, increasing to 24.4% identification at a false positive rate of 10% and 37.8% identification at a false positive rate of 15%. Taking the overlap of both early-onset pre-eclampsia outcome and severe pre-eclampsia outcome, screening by RBP4 was estimated to identify about 18% of individuals developing PeG34 at a false positive rate of 5%, increasing to 28% identification at a false positive rate of 10% and 42% detection at a false positive rate of 15%.

| False Positive Rate | Detection Rate Pe34 | Detection Rate PeG | Detection Rate PeG34 |
|---|---|---|---|
| 5% | 15.3% | 17.8% | 18% |
| 10% | 23.7% | 24.4% | 28% |
| 15% | 35.6% | 37.8% | 42% |

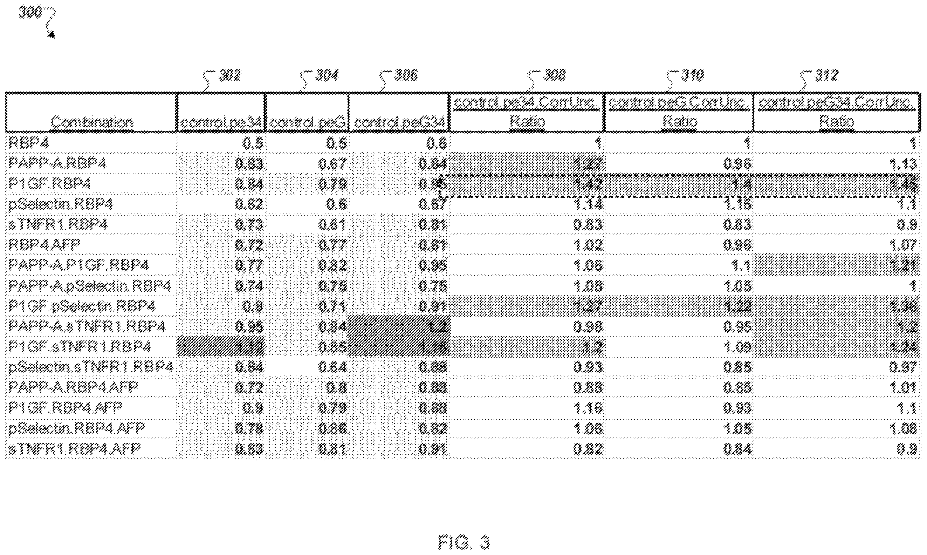

By combining analysis of measurements of RBP4 with measurements of one or more of PAPP-A, a p-Selectin, PlGF, sTNFR1, and AFP, detection rate may be further improved. Turning to FIG. 3, a table 300 demonstrates Mahalanobis distances between control and case (e.g., Pe34, PeG, and PeG34) groups for various combinations of RBP4 plus one or more of PAPP-A, a p-Selectin, PlGF, sTNFR1, and AFP, as well as analysis showing increased "over the sum" -effect for selected combinations. In the first three result analysis columns 302, 304, and 306, Mahalanobis distances between control and case groups are listed, with distances 0.7 of greater highlighted in light gray, and distances 1.0 and greater highlighted in dark gray. As can be seen in the table 300, combinations demonstrating notably favorable performance include RBP4 plus PlGF plus sTNFR1, having a Mahalonobis distance of 1.12 for Pe34 outcome, 0.85 for PeG outcome, and 1.16 for PeG34 outcome, as well as the combination of RBP4 plus PAPP-A plus sTNFR1, having a Mahalonobis distance of 0.95 for Pe34 outcome, 0.84 for PeG outcome, and 1.2 for PeG34 outcome.

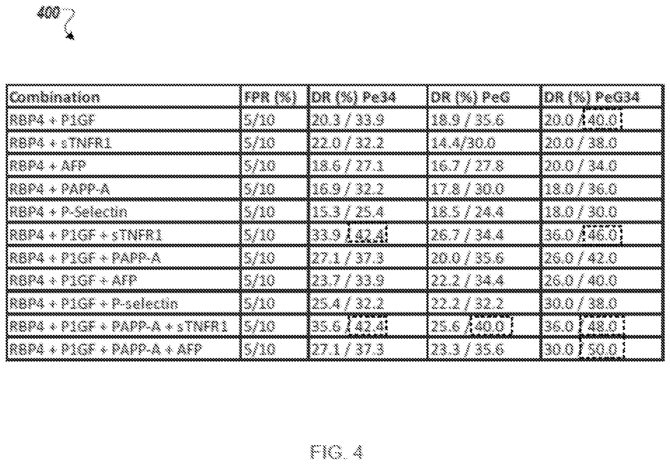

Turning to FIG. 4, a table 400 lists corresponding detection rates for the various combinations of table 300 of FIG. 3, including detection rates correlated to a false positive rate of both 5% and 10%. Compared to a detection rate of RBP4 alone, having a 15.3% detection rate with about a 5% false positive rate and a 23.7% detection rate with about a 10% false positive rate, each combination demonstrates a benefit. As highlighted within the table 400, a particularly beneficial combination in relation to detection of early onset pre-eclampsia (Pe34) appears to be RBP4 plus PlGF plus sTNFR1, having a detection rate of 33.9 at a false positive rate of 5%, and a detection rate of 42.4 at a false positive rate of 10%, as well as the combination of RBP4 plus PlGF plus PAPP-A plus sTNFR1, having a detection rate of 35.6% at a false positive rate of 5% and a detection rate of 42.4% at a false positive rate of 10%. Highlighted in relation to severe pre-eclampsia (PeG), a combination of RBP4 plus PlGF plus PAPP-A plus sTNFR1 demonstrates a detection rate of 25.6% at a false positive rate of 5% and a detection rate of 40.0% at a false positive rate of 10%. In considering detection of risk of the combination of severe early-onset pre-eclampsia (PeG34), four separate combinations are highlighted: the combination of RBP4 plus PlGF, having a detection rate of 20.0% at a false positive rate of 5% and a detection rate of 40.0% at a false positive rate of 10%; the combination of RBP4 plus PlGF plus sTNFR1, having a detection rate of 36.0% at a false positive rate of 5% and a detection rate of 46.0% at a false positive rate of 10%; the combination of RBP4 plus PlGF plus PAPP-A plus sTNFR1, having a detection rate of 36.0% at a false positive rate of 5% and a detection rate of 48.0% at a false positive rate of 10%; and the combination of RBP4 plus PlGF plus PAPP-A plus AFP, having a detection rate of 30.0% at a false positive rate of 5% and a detection rate of 50.0% at a false positive rate of 10%.

In some implementations, a synergistic benefit may be obtained with combined analysis including the RBP4 biochemical marker and one or more additional biochemical markers, for example selected from the following: PlGF, P-selectin, sP-selectin, PAPP-A, AFP, and sTNFR1. As illustrated in columns 308, 310, and 312 of FIG. 3, for example, ratios of the Mahalanobis distance of correlated results vs. Mahalanobis distances expected based upon individual Mahalanobis differences (e.g., calculated taking into account only the variances observed in each individual marker) are presented. The Mahalanobis correlated to uncorrelated ratios may be reviewed to identify combinations of biochemical markers demonstrating particularly synergistic benefits. Particularly promising combinations, as identified based upon the Mahalanobis correlated to uncorrelated ratios, are highlighted in gray in each of columns 308, 310, and 312. Furthermore, the combination of PlGF plus RBP4, having a Mahalanobis correlated to uncorrelated ratio of 1.42 / 1.4 /1.45 in relation to Pe34 / PeG / PeG32 is circled as being identified as a particularly synergistic combination, based upon the Mahalanobis correlated to uncorrelated ratio calculations.

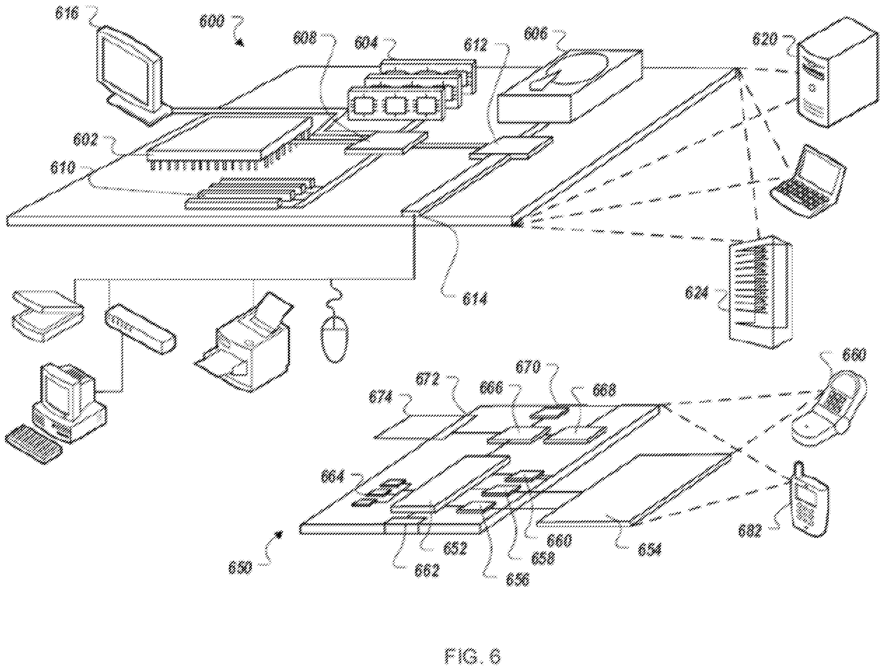

FIG. 6 shows an example of a computing device 600 and a mobile computing device 650 that can be used to implement the techniques described in this disclosure. The computing device 600 is intended to represent various forms of digital computers, such as laptops, desktops, workstations, personal digital assistants, servers, blade servers, mainframes, and other appropriate computers. The mobile computing device 650 is intended to represent various forms of mobile devices, such as personal digital assistants, cellular telephones, smart-phones, and other similar computing devices. The components shown here, their connections and relationships, and their functions, are meant to be examples only, and are not meant to be limiting.

The computing device 600 includes a processor 602, a memory 604, a storage device 606, a high-speed interface 608 connecting to the memory 604 and multiple high-speed expansion ports 610, and a low-speed interface 612 connecting to a low-speed expansion port 614 and the storage device 606. Each of the processor 602, the memory 604, the storage device 606, the high-speed interface 608, the high-speed expansion ports 610, and the low-speed interface 612, are interconnected using various busses, and may be mounted on a common motherboard or in other manners as appropriate. The processor 602 can process instructions for execution within the computing device 600, including instructions stored in the memory 604 or on the storage device 606 to display graphical information for a GUI on an external input/output device, such as a display 616 coupled to the high-speed interface 608. In other implementations, multiple processors and/or multiple buses may be used, as appropriate, along with multiple memories and types of memory. Also, multiple computing devices may be connected, with each device providing portions of the necessary operations (e.g., as a server bank, a group of blade servers, or a multi-processor system).

The memory 604 stores information within the computing device 600. In some implementations, the memory 604 is a volatile memory unit or units. In some implementations, the memory 604 is a non-volatile memory unit or units. The memory 604 may also be another form of computer-readable medium, such as a magnetic or optical disk.

The storage device 606 is capable of providing mass storage for the computing device 600. In some implementations, the storage device 606 may be or contain a computer-readable medium, such as a floppy disk device, a hard disk device, an optical disk device, or a tape device, a flash memory or other similar solid state memory device, or an array of devices, including devices in a storage area network or other configurations. Instructions can be stored in an information carrier. The instructions, when executed by one or more processing devices (for example, processor 602), perform one or more methods, such as those described above. The instructions can also be stored by one or more storage devices such as computer- or machine-readable mediums (for example, the memory 604, the storage device 606, or memory on the processor 602).

The high-speed interface 608 manages bandwidth-intensive operations for the computing device 600, while the low-speed interface 612 manages lower bandwidth-intensive operations. Such allocation of functions is an example only. In some implementations, the high-speed interface 608 is coupled to the memory 604, the display 616 (e.g., through a graphics processor or accelerator), and to the high-speed expansion ports 610, which may accept various expansion cards (not shown). In the implementation, the low-speed interface 612 is coupled to the storage device 606 and the low-speed expansion port 614. The low-speed expansion port 614, which may include various communication ports (e.g., USB, Bluetooth®, Ethernet, wireless Ethernet) may be coupled to one or more input/output devices, such as a keyboard, a pointing device, a scanner, or a networking device such as a switch or router, e.g., through a network adapter.

The computing device 600 may be implemented in a number of different forms, as shown in the figure. For example, it may be implemented as a standard server 620, or multiple times in a group of such servers. In addition, it may be implemented in a personal computer such as a laptop computer 622. It may also be implemented as part of a rack server system 624. Alternatively, components from the computing device 600 may be combined with other components in a mobile device (not shown), such as a mobile computing device 650. Each of such devices may contain one or more of the computing device 600 and the mobile computing device 650, and an entire system may be made up of multiple computing devices communicating with each other.

The mobile computing device 650 includes a processor 652, a memory 664, an input/output device such as a display 654, a communication interface 666, and a transceiver 668, among other components. The mobile computing device 650 may also be provided with a storage device, such as a micro-drive or other device, to provide additional storage. Each of the processor 652, the memory 664, the display 654, the communication interface 666, and the transceiver 668, are interconnected using various buses, and several of the components may be mounted on a common motherboard or in other manners as appropriate.

The processor 652 can execute instructions within the mobile computing device 650, including instructions stored in the memory 664. The processor 652 may be implemented as a chipset of chips that include separate and multiple analog and digital processors. The processor 652 may provide, for example, for coordination of the other components of the mobile computing device 650, such as control of user interfaces, applications run by the mobile computing device 650, and wireless communication by the mobile computing device 650.

The processor 652 may communicate with a user through a control interface 658 and a display interface 656 coupled to the display 654. The display 654 may be, for example, a TFT (Thin-Film-Transistor Liquid Crystal Display) display or an OLED (Organic Light Emitting Diode) display, or other appropriate display technology. The display interface 656 may include appropriate circuitry for driving the display 654 to present graphical and other information to a user. The control interface 658 may receive commands from a user and convert them for submission to the processor 652. In addition, an external interface 662 may provide communication with the processor 652, so as to enable near area communication of the mobile computing device 650 with other devices. The external interface 662 may provide, for example, for wired communication in some implementations, or for wireless communication in other implementations, and multiple interfaces may also be used.

The memory 664 stores information within the mobile computing device 650. The memory 664 can be implemented as one or more of a computer-readable medium or media, a volatile memory unit or units, or a non-volatile memory unit or units. An expansion memory 674 may also be provided and connected to the mobile computing device 650 through an expansion interface 672, which may include, for example, a SIMM (Single In Line Memory Module) card interface. The expansion memory 674 may provide extra storage space for the mobile computing device 650, or may also store applications or other information for the mobile computing device 650. Specifically, the expansion memory 674 may include instructions to carry out or supplement the processes described above, and may include secure information also. Thus, for example, the expansion memory 674 may be provide as a security module for the mobile computing device 650, and may be programmed with instructions that permit secure use of the mobile computing device 650. In addition, secure applications may be provided via the SIMM cards, along with additional information, such as placing identifying information on the SIMM card in a non-hackable manner.

The memory may include, for example, flash memory and/or NVRAM memory (non-volatile random access memory), as discussed below. In some implementations, instructions are stored in an information carrier. The instructions, when executed by one or more processing devices (for example, processor 652), perform one or more methods, such as those described above. The instructions can also be stored by one or more storage devices, such as one or more computer- or machine-readable mediums (for example, the memory 664, the expansion memory 674, or memory on the processor 652). In some implementations, the instructions can be received in a propagated signal, for example, over the transceiver 668 or the external interface 662.

The mobile computing device 650 may communicate wirelessly through the communication interface 666, which may include digital signal processing circuitry where necessary. The communication interface 666 may provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication may occur, for example, through the transceiver 668 using a radio-frequency. In addition, short-range communication may occur, such as using a Bluetooth®, Wi-Fi™, or other such transceiver (not shown). In addition, a GPS (Global Positioning System) receiver module 670 may provide additional navigation- and location-related wireless data to the mobile computing device 650, which may be used as appropriate by applications running on the mobile computing device 650.

The mobile computing device 650 may also communicate audibly using an audio codec 660, which may receive spoken information from a user and convert it to usable digital information. The audio codec 660 may likewise generate audible sound for a user, such as through a speaker, e.g., in a handset of the mobile computing device 650. Such sound may include sound from voice telephone calls, may include recorded sound (e.g., voice messages, music files, etc.) and may also include sound generated by applications operating on the mobile computing device 650.

The mobile computing device 650 may be implemented in a number of different forms, as shown in the figure. For example, it may be implemented as a cellular telephone 680. It may also be implemented as part of a smart-phone 682, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

In view of the structure, functions and apparatus of the systems and methods described here, in some implementations, a systems, methods, and apparatus for identifying risk of a pregnant individual in having or developing pre-eclampsia, including one or both of early-onset pre-eclampsia and severe pre-eclampsia, are provided. Having described certain implementations of methods, systems, and apparatus for supporting assessment of risk of a pregnant individual in having or developing pre-eclampsia, including one or both of early-onset pre-eclampsia and severe pre-eclampsia, it will now become apparent to one of skill in the art that other implementations incorporating the concepts of the disclosure may be used.

## Claims

1. A method for predicting risk of pre-eclampsia in a pregnant individual, the method comprising:
measuring biochemical markers including an RBP4 biochemical marker and a PlGF biochemical marker in a blood sample obtained from the pregnant individual to determine biomarker levels including an RBP4 biomarker level and a PlGF biomarker level;
identifying, by a processor of a computing device, for each of the measured biochemical markers, a difference between the measured biomarker level and a corresponding predetermined control level; and
responsive to the identifying, determining, by the processor, a prediction corresponding to a relative risk of the pregnant individual developing pre-eclampsia.

2. The method according to claim 1, wherein measuring the biochemical markers further comprises measuring one or more of a P-Selectin biochemical marker, a PAPP-A biochemical marker, an AFP biochemical marker, and an sTNFR1 biochemical marker.

3. The method according to claim 1 or 2, wherein the prediction corresponds to a relative risk of the pregnant individual developing:
i) early-onset pre-eclampsia (Pe34); and/or
ii) at least one of severe pre-eclampsia (PeG) and severe early-onset pre-eclampsia (PeG34).

4. The method according to any of the preceding claims, wherein the difference comprises at least one of a threshold value and a percentage difference.

5. The method according to any of the preceding claims, wherein the prediction is based in part upon at least one maternal history factor of the pregnant individual; optionally wherein the at least one maternal history factor comprises one of a gestational age, a weight, a BMI, a family history status, an ethnicity, and a smoking status.

6. The method according to any of the preceding claims, wherein the prediction is positive based at least in part upon identifying the RBP4 biomarker level reflects a statistically significant increase in comparison to a respective control level.

7. The method according to any one of the preceding claims, wherein determining the prediction comprises calculating a risk assessment score optionally wherein the risk assessment score comprises a proportional risk value, further optionally, wherein the risk assessment score comprises a numeric risk score assigned on a scale.

8. The method according to any one of the preceding claims, wherein the pregnant individual is within a first trimester stage of pregnancy at time of obtaining the blood sample; optionally wherein the first trimester stage ranges from forty-two days from conception to ninety-seven days from conception.

9. The method according to any one of the preceding claims, wherein the blood sample comprises one of a plasma sample and a serum sample.

10. The method according to any one of the preceding claims, wherein measuring the biochemical markers comprises:
i) performing a quantitative immunoassay; and/or
ii) determining a concentration of each respective biochemical marker; and/or
iii) determining a quantity of each respective biochemical marker.

11. A method for predicting risk of pre-eclampsia in a pregnant individual, the method comprising:
measuring biochemical markers in a blood sample obtained from the pregnant individual to determine biomarker levels, wherein
a first biomarker of the biochemical markers comprises RBP4, and a first biomarker level comprises an RBP4 biomarker level, and a second biomarker of the biochemical markers comprises PlGF and a second biomarker level comprises a PlGF biomarker level; and
calculating, by the processor, a risk assessment score corresponding to a relative risk of the pregnant individual developing pre-eclampsia, wherein the risk assessment score is based at least in part upon the RBP4 biomarker level and the PlGF biomarker level.

12. The method according to claim 11, wherein the risk assessment score is based at least in part upon a comparison of the RBP4 biomarker level and a corresponding predetermined control level, and/or wherein measuring the biochemical markers further comprises:
measuring one or more of a P-Selectin biochemical marker, a PAPP-A biochemical marker, an AFP biochemical marker, and a sTNFR1 biochemical marker; and/or
applying mass spectrometry analysis; and/or
wherein calculating the risk assessment score comprises normalizing the comparison of the biomarker level and the corresponding predetermined control level based upon:
i) one or more maternal demographic values; optionally wherein normalizing the comparison comprises applying a multiple of mean statistical analysis; and/or
ii) one or more maternal biophysical attributes.

13. A system for predicting risk of pre-eclampsia in a pregnant individual comprising:
an in vitro diagnostics kit comprising testing instruments for testing a blood sample obtained from the pregnant individual for biochemical markers including an RBP4 biochemical marker and a PlGF biochemical marker; and
a non-transitory computer-readable medium having instructions stored thereon, wherein the instructions, when executed by a processor, cause the processor to;
retrieve biomarker levels, wherein each biomarker level corresponds to a biochemical marker tested for using the in vitro diagnostics kit, and wherein the retrieved biomarker levels comprises an RBP4 biomarker level and a PlGF biomarker level; and
calculate a risk assessment score corresponding to a relative risk of the pregnant individual developing pre-eclampsia, wherein the risk assessment score is based at least in part upon the RBP4 biomarker level and the PlGF biomarker level.

14. The system according to claim 13, wherein measuring the biochemical markers further comprises measuring one or more of a P-Selectin biochemical marker, a PAPP-A biochemical marker, an AFP biochemical marker, and a sTNFR1 biochemical marker, and/or
wherein the risk assessment score is based at least in part upon a comparison of the RBP4 biomarker level and a corresponding predetermined control level, and/or wherein the instructions cause the processor to:
i) prior to calculating the risk assessment score, access at least one maternal history factor of the pregnant individual; optionally wherein accessing the at least one maternal history factor of the pregnant individual comprises:
(a) causing presentation of a graphical user interface at a display device, wherein the graphical user interface comprises one or more input fields for submitting maternal history factor information regarding the pregnant individual; and/or
(b) importing, from an electronic medical record, the at least one maternal history factor.
ii) after calculating the risk assessment score, cause presentation of the risk assessment score at a display device; optionally
wherein causing presentation of the risk assessment score comprises causing presentation of risk assessment information, and/or wherein the testing instruments comprise an assay buffer, optionally wherein the testing instruments comprise one or more of a coated plate, a tracer, and calibrators.

15. A system for predicting risk of pre-eclampsia in a pregnant individual comprising:
an in vitro diagnostics kit comprising testing instruments for testing a blood sample obtained from the pregnant individual for biochemical markers, wherein
a first biomarker of the biochemical markers comprises RBP4;
a second biomarker of the biochemical markers comprises PlGF; and
a non-transitory computer-readable medium having instructions stored thereon, wherein the instructions, when executed by a processor, cause the processor to:
retrieve biomarker levels, wherein each biomarker level corresponds to a biochemical marker tested for using the in vitro diagnostics kit, and wherein the retrieved biomarker levels comprises an RBP4 biomarker level and a PlGF biomarker level; and
identify, for each of the one or more measured biochemical markers, a difference between the measured biomarker level and a corresponding predetermined control level; and
responsive to the identifying, determine a prediction corresponding to a relative risk of the pregnant individual developing pre-eclampsia.

16. The system according to claim 15, wherein the biomarkers comprise an additional biomarker, which is one of a P-Selectin biochemical marker, a PAPP-A biochemical marker, an AFP biochemical marker, and a sTNFR1 biochemical marker.

17. A non-transitory computer readable medium having instructions stored thereon, wherein the instructions, when executed by a processor, cause the processor to:
access measurements of biochemical markers, wherein
the measurements were obtained by testing biochemical marker levels in a blood sample obtained from a pregnant individual,
a first biomarker of the biochemical markers comprises an RBP4 biomarker, and
a second biomarker of the biochemical markers comprises PlGF level and a second biomarker level comprises a PlGF biomarker level, and
a first measurement comprises an RBP4 level, and
a second measurement comprises a PlGF level; and
calculate a risk assessment score corresponding to a relative risk of the pregnant individual developing pre-eclampsia, wherein the risk assessment score is based at least in part upon the RBP4 biomarker level and the PlGF biomarker level.

18. The non-transitory computer readable medium according to claim 17, wherein the biomarkers comprise an additional biomarker, which is one of a P-Selectin biochemical marker, a PAPPA biochemical marker, an AFP biochemical marker, and a sTNFR1 biochemical marker; in which case, optionally wherein the risk assessment score is based at least in part on a comparison of the RBP4 biomarker level and a corresponding predetermined control level.

19. A non-transitory computer readable medium having instructions stored thereon, wherein the instructions, when executed by a processor, cause the processor to:
access measurements of biochemical markers, wherein
the measurements were obtained by testing biochemical marker levels in a blood sample obtained from a pregnant individual, and
a first biomarker of the biochemical markers comprises RBP4, and
a second biomarker of the biochemical markers comprises PlGF;
identify, for each of the measured biochemical markers, a difference between the measured biomarker level and a corresponding predetermined control level; and
responsive to the identifying, determine a prediction corresponding to a relative risk of the pregnant individual developing pre-eclampsia.

20. The non-transitory computer readable medium according to claim 19, wherein the biomarkers comprise an additional biomarker, which is one of a P-Selectin biochemical marker, a PAPP-A biochemical marker, an AFP biochemical marker, and a sTNFR1 biochemical marker.

## Patentansprüche

1. Verfahren zum Vorhersagen eines Risikos von Präeklampsie in einem schwangeren Individuum, wobei das Verfahren Folgendes umfasst:
Messen von biochemischen Markern, einschließlich eines biochemischen RBP4-Markers und eines biochemischen PIGF-Markers, in einer Blutprobe, die von dem schwangeren Individuum entnommen wird, um Spiegel von Biomarkern, einschließlich eines Spiegels eines RBP4-Biomarkers und eines Spiegels eines PIGF-Biomarkers, zu bestimmen;
Identifizieren einer Differenz zwischen dem gemessenen Spiegel eines Biomarkers und einem entsprechenden vorbestimmten Kontrollspiegel für jeden der gemessenen biochemischen Marker durch einen Prozessor einer Rechenvorrichtung; und
als Reaktion auf das Identifizieren, Bestimmen einer Vorhersage durch den Prozessor, die einem relativen Risiko entspricht, dass das schwangere Individuum Präeklampsie entwickelt.

2. Verfahren nach Anspruch 1, wobei das Messen der biochemischen Marker ferner das Messen eines biochemischen P-Selectin-Markers, eines biochemischen PAPP-A-Markers, eines biochemischen AFP-Markers oder eines biochemischen sTNFR1-Markers umfasst.

3. Verfahren nach Anspruch 1 oder 2, wobei die Vorhersage einem relativen Risiko entspricht, dass das schwangere Individuum Folgendes entwickelt:
i) früh auftretende Präeklampsie (Pe34); und/oder
ii) eine schwere Präeklampsie (PeG) und/oder eine schwere früh auftretende Präeklampsie (PeG34).

4. Verfahren nach einem der vorhergehenden Ansprüche, wobei die Differenz einen Schwellenwert und/oder eine prozentuale Differenz umfasst.

5. Verfahren nach einem der vorhergehenden Ansprüche, wobei die Vorhersage teilweise auf wenigstens einem Faktor der mütterlichen Krankengeschichte des schwangeren Individuums basiert;
optional wobei der wenigstens eine Faktor der mütterlichen Krankengeschichte ein Schwangerschaftsalter, ein Gewicht, einen BMI, einen Status der familiären Krankengeschichte, eine ethnische Zugehörigkeit und einen Raucherstatus umfasst.

6. Verfahren nach einem der vorhergehenden Ansprüche, wobei die Vorhersage wenigstens teilweise darauf basiert, dass das Identifizieren des Spiegels des RBP4-Biomarkers einen statistisch bedeutenden Zuwachs im Vergleich zu einem jeweiligen Kontrollspiegel widerspiegelt.

7. Verfahren nach einem der vorhergehenden Ansprüche, wobei das Bestimmen der Vorhersage das Berechnen eines Risikobewertungsskalenwerts umfasst, optional wobei der Risikobewertungsskalenwert einen proportionalen Risikowert umfasst, ferner optional wobei der Risikobewertungsskalenwert einen numerischen Risikoskalenwert umfasst, der auf einer Skala zugewiesen ist.

8. Verfahren nach einem der vorhergehenden Ansprüche, wobei sich das schwangere Individuum zu dem Zeitpunkt des Entnehmens der Blutprobe innerhalb eines ersten Trimesterschwangerschaftsstadiums befindet;
optional wobei das erste Trimesterstadium in einem Bereich von zweiundvierzig Tagen von der Empfängnis bis siebenundneunzig Tagen von der Empfängnis liegt.

9. Verfahren nach einem der vorhergehenden Ansprüche, wobei die Blutprobe eine Plasmaprobe oder eine Serumprobe umfasst.

10. Verfahren nach einem der vorhergehenden Ansprüche, wobei das Messen der biochemischen Marker Folgendes umfasst:
i) Durchführen eines quantitativen Immunassays; und/oder
ii) Bestimmen einer Konzentration jedes jeweiligen biochemischen Markers; und/oder
iii) Bestimmen einer Menge jedes jeweiligen biochemischen Markers.

11. Verfahren zum Vorhersagen eines Risikos von Präeklampsie in einem schwangeren Individuum, wobei das Verfahren Folgendes umfasst:
Messen von biochemischen Markern in einer Blutprobe, die von dem schwangeren Individuum entnommen wird, um Spiegel eines Biomarkers zu bestimmen, wobei ein erster Biomarker der biochemischen Marker RBP4 umfasst und ein erster Spiegel eines Biomarkers einen Spiegel des RBP4-Biomarkers umfasst und ein zweiter Biomarker der biochemischen Marker PIGF umfasst und ein zweiter Spiegel eines Biomarkers einen Spiegel eines PIGF-Biomarkers umfasst; und
Berechnen eines Risikobewertungsskalenwerts, der einem relativen Risiko entspricht, dass das schwangere Individuum eine Präeklampsie entwickelt, durch den Prozessor, wobei der Risikobewertungsskalenwert wenigstens teilweise auf dem Spiegel des RBP4-Biomarkers und dem Spiegel des PIGF-Biomarkers basiert.

12. Verfahren nach Anspruch 11, wobei der Risikobewertungsskalenwert wenigstens teilweise auf einem Vergleich des Spiegels des RBP4-Biomarkers und einem entsprechenden vorbestimmten Kontrollspiegel basiert, und/oder wobei das Messen der biochemischen Marker ferner Folgendes umfasst:
Messen eines biochemischen P-Selectin-Markers, eines biochemischen PAPP-A-Markers, eines biochemischen AFP-Markers oder eines biochemischen sTNFR1-Markers; und/oder
Anwenden einer Massenspektrometrieanalyse; und/oder
wobei das Berechnen der Risikobewertungsskalenwerts ein Normalisieren des Vergleichs des Spiegels eines Biomarkers und des entsprechenden vorbestimmten Kontrollspiegels umfasst, basierend auf Folgendem:
i) einem oder mehreren demografischen Werten der Mutter; optional wobei das Normalisieren des Vergleichs das Anwenden eines Vielfachen der mittleren statistischen Analyse umfasst; und/oder
ii) einem oder mehreren biophysikalischen Attributen der Mutter.

13. System zum Vorhersagen eines Risikos einer Präeklampsie in einem schwangeren Individuum, Folgendes umfassend:
ein *In*-*vitro*-Diagnostikkit, umfassend Testinstrumente zum Testen einer von dem schwangeren Individuum entnommenen Blutprobe auf biochemische Marker, einschließlich eines biochemischen RBP4-Markers und eines biochemischen PIGF-Markers; und
ein nichtflüchtiges computerlesbares Medium mit darauf gespeicherten Anweisungen, wobei die Anweisungen, wenn sie durch einen Prozessor ausgeführt werden, den Prozessor zu Folgendem veranlassen;
Abrufen von Spiegeln eines Biomarkers, wobei jeder Spiegel eines Biomarkers einem biochemischen Marker entspricht, auf den unter Verwendung des *In*-*vitro*-Diagnostikkits getestet wird, und wobei die abgerufenen Spiegel eines Biomarkers einen Spiegel eines RBP4-Biomarkers und einen Spiegel eines PIGF-Biomarkers umfassen; und
Berechnen eines Risikobewertungsskalenwerts, der einem relativen Risiko entspricht, dass das schwangere Individuum eine Präeklampsie entwickelt, wobei der Risikobewertungsskalenwert wenigstens teilweise auf dem Spiegel des RBP4-Biomarkers und dem Spiegel des PIGF-Biomarkers basiert.

14. System nach Anspruch 13, wobei ferner das Messen der biochemischen Marker ferner das Messen eines biochemischen P-Selectin-Markers, eines biochemischen PAPP-A-Markers, eines biochemischen AFP-Markers oder eines biochemischen sTNFR1-Markers umfasst; und/oder
wobei der Risikoauswertungsskalenwert wenigstens teilweise auf einem Vergleich des Spiegels eines RBP4-Biomarkers und eines entsprechenden vorbestimmten Kontrollspiegels basiert und/oder wobei die Anweisungen den Prozessor zu Folgendem veranlassen:
i) vor dem Berechnen des Risikoauswertungsskalenwerts, Zugreifen auf wenigstens einen Faktor der mütterlichen Krankheitsgeschichte des schwangeren Individuums; optional wobei das Zugreifen auf den wenigstens einen Faktor der mütterlichen Krankheitsgeschichte des schwangeren Individuums Folgendes umfasst:
(a) Bewirken einer Präsentation einer grafischen Benutzerschnittstelle auf einer Anzeigevorrichtung, wobei die grafische Benutzerschnittstelle ein oder mehrere Eingabefelder zum Eintragen von Informationen bezüglich des Faktors der mütterlichen Krankengeschichte hinsichtlich des schwangeren Individuums; und/oder
(b) Importieren des wenigstens einen Faktors der mütterlichen Krankheitsgeschichte von einer elektronischen Krankenakte.
ii) nach dem Berechnen des Risikoauswertungsskalenwert, Bewirken einer Präsentation des Risikoauswertungsskalenwert auf einer Anzeigevorrichtung; optional wobei das Bewirken einer Präsentation des Riskoauswertungsskalenwerts das Bewirken einer Präsentation von Risikoauswertungsinformationen umfasst, und/oder wobei die Testinstrumente einen Assaypuffer umfassen, optional wobei die Testinstrumente eine beschichtete Platte, einen Tracer und/oder Kalibratoren umfasst.

15. System zum Vorhersagen eines Risikos einer Präeklampsie in einem schwangeren Individuum, Folgendes umfassend:
*In*-*vitro*-Diagnostikkit, umfassend Testinstrumente zum Testen einer von dem schwangeren Individuum entnommenen Blutprobe auf biochemische Marker, wobei ein erster Biomarker der biochemischen Marker RBP4 umfasst;
ein zweiter Biomarker der biochemischen Marker PIGF umfasst; und
ein nichtflüchtiges computerlesbares Medium mit darauf gespeicherten Anweisungen, wobei die Anweisungen, wenn sie durch einen Prozessor ausgeführt werden, den Prozessor zu Folgendem veranlassen:
Abrufen von Spiegeln eines Biomarkers, wobei jeder Spiegel eines Biomarkers einem biochemischen Marker entspricht, auf den unter Verwendung des *In*-*vitro*-Diagnostikkits getestet wird, und wobei die abgerufenen Spiegel eines Biomarkers einen Spiegel eines RBP4-Biomarkers und einen Spiegel eines PIGF-Biomarkers umfassen; und
Identifizieren einer Differenz zwischen dem gemessenen Spiegel eines Biomarkers und einem entsprechenden vorbestimmten Kontrollspiegel für jeden der einen oder der mehreren gemessenen biochemischen Marker; und
als Reaktion auf das Identifizieren, Bestimmen einer Vorhersage, die einem relativen Risiko entspricht, dass das schwangere Individuum Präeklampsie entwickelt.

16. System nach Anspruch 15, wobei die Biomarker einen zusätzlichen Biomarker umfassen, der ein biochemischer P-Selectin-Marker, ein biochemischer PAPP-A-Marker, ein biochemischer AFP-Markers oder ein biochemischer sTNFR1-Markers ist.

17. Nichtflüchtiges computerlesbares Medium mit darauf gespeicherten Anweisungen, wobei die Anweisungen, wenn sie durch einen Prozessor ausgeführt werden, den Prozessor zu Folgendem veranlassen:
Zugreifen auf Messungen von biochemischen Markern, wobei die Messungen durch das Testen von Spiegeln eines biochemischen Markers in einer Blutprobe erhalten werden, die von einem schwangeren Individuum entnommen wurde, wobei ein erster Biomarker der biochemischen Marker einen RBP4-Biomarker umfasst, und ein zweiter Biomarker der biochemischen Marker einen PIGF-Spiegel umfasst und ein zweiter Spiegel eines Biomarkers einen Spiegel eines PIGF-Biomarkers umfasst und eine erste Messung einen RBP4-Spiegel umfasst und eine zweite Messung einen PIGF-Spiegel umfasst; und
Berechnen eines Risikobewertungsskalenwerts, der einem relativen Risiko entspricht, dass das schwangere Individuum eine Präeklampsie entwickelt, wobei der Risikobewertungsskalenwert wenigstens teilweise auf dem Spiegel des RBP4-Biomarkers und dem Spiegel des PIGF-Biomarkers basiert.

18. Nichtflüchtiges computerlesbares Medium nach Anspruch 17, wobei die Biomarker einen zusätzlichen Biomarker umfassen, der ein biochemischer P-Selectin-Marker, ein biochemischer PAPP-A-Marker, ein biochemischer AFP-Markers oder ein biochemischer sTNFR1-Markers ist;
in diesem Fall, optional wobei der Risikoauswertungsskalenwert wenigstens teilweise auf einem Vergleich des Spiegels eines RBP4-Biomarkers und eines entsprechenden vorbestimmten Kontrollspiegel basiert.

19. Nichtflüchtiges computerlesbares Medium mit darauf gespeicherten Anweisungen, wobei die Anweisungen, wenn sie durch einen Prozessor ausgeführt werden, den Prozessor zu Folgendem veranlassen:
Zugreifen auf Messungen von biochemischen Markern, wobei die Messungen durch das Testen von Spiegeln eines biochemischen Markers in einer Blutprobe erhalten werden, die von einem schwangeren Individuum entnommen wurde, und ein erster Biomarker der biochemischen Marker RBP4 umfasst, und ein zweiter Biomarker der biochemischen Marker PIGF umfasst;
Identifizieren einer Differenz zwischen dem gemessenen Spiegel eines Biomarkers und einem entsprechenden vorbestimmten Kontrollspiegel für jeden gemessenen biochemischen Marker; und
als Reaktion auf das Identifizieren, Bestimmen einer Vorhersage, die einem relativen Risiko entspricht, dass das schwangere Individuum Präeklampsie entwickelt.

20. Nichtflüchtiges computerlesbares Medium nach Anspruch 19, wobei die Biomarker einen zusätzlichen Biomarker umfassen, der ein biochemischer P-Selectin-Marker, ein biochemischer PAPP-A-Marker, ein biochemischer AFP-Marker oder ein biochemischer sTNFR1-Marker ist.

## Revendications

1. Méthode de prédiction du risque de pré-éclampsie chez une femme enceinte, la méthode comprenant:
la mesure de marqueurs biochimiques dont un marqueur biochimique RBP4 et un marqueur biochimique PIGF dans un échantillon sanguin prélevé chez la femme enceinte afin de déterminer des niveaux de biomarqueurs, dont un niveau de biomarqueur RBP4 et un niveau de biomarqueur PIGF ;
l'identification, par un processeur d'un dispositif informatique, pour chacun des marqueurs biochimiques mesurés, d'une différence entre le niveau de biomarqueur mesuré et un niveau témoin prédéterminé correspondant ; et,
en réponse à l'identification, la détermination, par le processeur, d'une prédiction correspondant à un risque relatif que la femme enceinte développe une pré-éclampsie.

2. Méthode selon la revendication 1, dans lequel la mesure des marqueurs biochimiques comprend en outre la mesure d'un ou plusieurs des éléments que sont un marqueur biochimique P-sélectine, un marqueur biochimique PAPP-A, un marqueur biochimique AFP et un marqueur biochimique sTNFR1.

3. Méthode selon la revendication 1 ou 2, dans lequel la prédiction correspond à un risque relatif que la femme enceinte développe :
i) une pré-éclampsie à apparition précoce (Pe34) ; et/ou
ii) au moins une pré-éclampsie sévère (PeG) ou une pré-éclampsie sévère à apparition précoce (PeG34).

4. Méthode selon l'une des revendications précédentes, dans laquelle la différence comprend au moins une valeur seuil ou une différence en pourcentage.

5. Méthode selon l'une des revendications précédentes, dans laquelle la prédiction est basée en partie sur au moins un facteur d'antécédents maternels de la femme enceinte ;
éventuellement dans laquelle ledit facteur d'antécédents maternels comprend l'un des éléments que sont l'âge de gestation, le poids, l'IMC, le statut d'antécédents familiaux, l'ethnicité, et le statut tabagique.

6. Méthode selon l'une des revendications précédentes, dans laquelle la prédiction est positive d'après, au moins en partie, l'identification du niveau de biomarqueur RBP4 qui reflète une augmentation statistiquement significative par rapport à un niveau témoin respectif.

7. Méthode selon l'une des revendications précédentes, dans laquelle la détermination de la prédiction comprend le calcul d'un score d'évaluation de risques, éventuellement dans laquelle le score d'évaluation de risques comprend une valeur de risque proportionnelle, éventuellement en outre, dans laquelle le score d'évaluation de risques comprend un score de risque numérique attribué selon une échelle.

8. Méthode selon l'une des revendications précédentes, dans laquelle la femme enceinte est à l'étape du premier trimestre de la grossesse au moment du prélèvement de l'échantillon sanguin ;
éventuellement dans laquelle le premier trimestre est compris entre quarante-deux jours à partir de la conception à quatre-vingt-dix-sept jours à partir de la conception.

9. Méthode selon l'une des revendications précédentes, dans laquelle l'échantillon sanguin comprend un échantillon de plasma ou un échantillon de sérum.

10. Méthode selon l'une des revendications précédentes, dans laquelle la mesure des marqueurs biochimiques comprend :
i) la réalisation d'un dosage immunologique quantitatif ; et/ou
ii) la détermination d'une concentration de chaque marqueur biochimique respectif ; et/ou
iii) la détermination d'une quantité de chaque marqueur biochimique respectif.

11. Méthode de prédiction du risque de pré-éclampsie chez une femme enceinte, la méthode comprenant :
la mesure de marqueurs biochimiques dans un échantillon sanguin prélevé chez la femme enceinte afin de déterminer des niveaux de biomarqueurs, dans laquelle un premier biomarqueur de marqueurs biochimiques comprend du RBP4, un premier niveau de biomarqueur comprend un niveau de biomarqueur RBP4, un second biomarqueur des marqueurs biochimiques comprend du PIGF, et un second niveau de biomarqueur comprend un niveau de biomarqueur PIGF ; et
le calcul, par le processeur, d'un score d'évaluation de risques correspondant à un risque relatif que la femme enceinte développe une pré-éclampsie, dans laquelle le score d'évaluation de risques est basé au moins en partie sur le niveau de biomarqueur RBP4 et le niveau de biomarqueur PIGF.

12. Méthode selon la revendication 11, dans laquelle le score d'évaluation de risques est basé au moins en partie sur une comparaison du niveau de biomarqueur RBP4 à un niveau témoin prédéterminé correspondant, et/ou dans laquelle la mesure des marqueurs biochimiques comprend en outre :
la mesure d'un ou plusieurs des éléments que sont un marqueur biochimique P-sélectine, un marqueur biochimique PAPP-A, un marqueur biochimique AFP et un marqueur biochimique sTNFR1 ; et/ou
l'application d'une analyse par spectrométrie de masse ; et/ou
dans laquelle le calcul du score d'évaluation de risques comprend la normalisation de la comparaison du niveau de biomarqueur et du niveau témoin prédéterminé correspondant d'après :
i) une ou plusieurs valeurs démographiques sur la maternité ; éventuellement dans laquelle la normalisation de la comparaison comprend l'application de plusieurs analyses statistiques moyennes ; et/ou
ii) un ou plusieurs attributs biophysiques maternels.

13. Système de prédiction du risque de pré-éclampsie chez une femme enceinte, comprenant :
un kit de diagnostic in vitro comprenant des instruments de test permettant de tester un échantillon sanguin prélevé chez la femme enceinte pour des marqueurs biochimiques, dont un marqueur biochimique RBP4 et un marqueur biochimique PIGF ; et
un support non transitoire lisible par ordinateur, sur lequel sont stockées des instructions qui, lorsqu'elles sont exécutées par un processeur, amènent le processeur à :
récupérer des niveaux de biomarqueurs, chaque niveau de biomarqueur correspondant à un marqueur biochimique testé pour l'utilisation du kit de diagnostic in vitro, et dans lequel les niveaux de biomarqueurs récupérés comprennent un niveau de biomarqueur RBP4 et un niveau de biomarqueur PIGF ; et à
calculer un score d'évaluation de risques correspondant à un risque relatif que la femme enceinte développe une pré-éclampsie, le score d'évaluation de risques étant basé au moins en partie sur le niveau de biomarqueur RBP4 et le niveau de biomarqueur PIGF.

14. Système selon la revendication 13, dans lequel la mesure des marqueurs biochimiques comprend en outre la mesure d'un ou de plusieurs des éléments que sont un marqueur biochimique P-sélectine, un marqueur biochimique PAPP-A, un marqueur biochimique AFP et un marqueur biochimique sTNFR1, et/ou
dans lequel le score d'évaluation de risques est basé au moins en partie sur une comparaison du niveau de biomarqueur RBP4 et d'un niveau témoin prédéterminé correspondant, et/ou dans lequel les instructions amènent le processeur à :
i) accéder, avant le calcul du score d'évaluation de risques, à au moins un facteur d'antécédents maternels de la femme enceinte ; éventuellement dans lequel l'accès audit facteur d'antécédents maternels de la femme enceinte comprend :
(a) la présentation d'une interface utilisateur graphique sur un dispositif d'affichage, l'interface utilisateur graphique comprenant un ou plusieurs champs d'entrée permettant de communiquer des informations sur le facteur d'antécédents maternels concernant la femme enceinte ; et/ou
(b) l'importation, à partir d'un dossier médical électronique, dudit facteur d'antécédents maternels,
ii) présenter, après avoir calculé le score d'évaluation de risques, le score d'évaluation de risques sur un dispositif d'affichage ;
éventuellement dans lequel la présentation du score d'évaluation de risques comprend la présentation d'informations d'évaluation de risques, et/ou dans lequel les instruments de test comprennent un tampon de dosage, éventuellement dans lequel les instruments de test comprennent un ou plusieurs éléments que sont une plaque revêtue, un traceur et des dispositifs d'étalonnage.

15. Système de prédiction du risque de pré-éclampsie chez une femme enceinte, comprenant :
un kit de diagnostic in vitro comprenant des instruments de test permettant de tester un échantillon sanguin prélevé chez la femme enceinte pour des marqueurs biochimiques, dans lequel un premier biomarqueur des marqueurs biochimiques comprend du RBP4 ;
dans lequel un second biomarqueur des marqueurs biochimiques comprend du PIGF ; et
un support non transitoire lisible par ordinateur, sur lequel sont stockées des instructions qui, lorsqu'elles sont exécutées par un processeur, amènent le processeur à :
récupérer des niveaux de biomarqueurs, chaque niveau de biomarqueur correspondant à un marqueur biochimique testé pour l'utilisation du kit de diagnostic in vitro, et dans lequel les niveaux de biomarqueurs récupérés comprennent un niveau de biomarqueur RBP4 et un niveau de biomarqueur PIGF ; et à
identifier, pour chacun des marqueurs biochimiques mesurés, une différence entre le niveau de biomarqueur mesuré et un niveau témoin prédéterminé correspondant ; et à
déterminer, en réponse à l'identification, une prédiction correspondant à un risque relatif qu'une femme enceinte développe une pré-éclampsie.

16. Système selon la revendication 15, dans lequel les biomarqueurs comprennent un biomarqueur supplémentaire, qui est l'un des éléments que sont un marqueur biochimique P-sélectine, un marqueur biochimique PAPP-A, un marqueur biochimique AFP et un marqueur biochimique sTNFR1.

17. Support non transitoire lisible par ordinateur, sur lequel sont stockées des instructions qui, lorsqu'elles sont exécutées par un processeur, amènent le processeur à :
accéder à des mesures de marqueurs biochimiques, lesdites mesures ayant été obtenues par un test des niveaux de marqueurs biochimiques dans un échantillon sanguin prélevé chez une femme enceinte, un premier biomarqueur des marqueurs biochimiques comprenant un biomarqueur RBP4, un second biomarqueur des marqueurs biochimiques comprenant un niveau de PIGF, un second niveau de biomarqueur comprenant un niveau de biomarqueur PIGF, une première mesure comprenant un niveau de RBP4, et une seconde mesure comprenant un niveau de PIGF ; et à
calculer un score d'évaluation de risques correspondant à un risque relatif que la femme enceinte développe une pré-éclampsie, le score d'évaluation de risques étant basé au moins en partie sur le niveau de biomarqueur RBP4 et le niveau de biomarqueur PIGF.

18. Support non transitoire lisible par ordinateur selon la revendication 17, dans lequel les biomarqueurs comprennent un biomarqueur supplémentaire, qui est l'un des éléments que sont un marqueur biochimique P-sélectine, un marqueur biochimique PAPPA, un marqueur biochimique AFP et un marqueur biochimique sTNFR1 ;
auquel cas, éventuellement, le score d'évaluation de risques est basé au moins en partie sur une comparaison du niveau de biomarqueur RBP4 à un niveau témoin prédéterminé correspondant.

19. Support non transitoire lisible par ordinateur, sur lequel sont stockées des instructions qui, lorsqu'elles sont exécutées par un processeur, amènent le processeur à :
accéder à des mesures de marqueurs biochimiques, lesdites mesures ayant été obtenues par un test des niveaux de marqueurs biochimiques dans un échantillon sanguin prélevé chez une femme enceinte, un premier biomarqueur des marqueurs biochimiques comprenant du RBP4, et un second biomarqueur des marqueurs biochimiques comprenant du PIGF ;
identifier, pour chacun des marqueurs biochimiques mesurés, une différence entre le niveau de biomarqueur mesuré et un niveau témoin prédéterminé correspondant ; et à
déterminer, en réponse à l'identification, une prédiction correspondant à un risque relatif qu'une femme enceinte développe une pré-éclampsie.

20. Support non transitoire lisible par ordinateur selon la revendication 19, dans lequel les biomarqueurs comprennent un biomarqueur supplémentaire, qui est l'un des éléments que sont un marqueur biochimique P-sélectine, un marqueur biochimique PAPP-A, un marqueur biochimique AFP et un marqueur biochimique sTNFR1.
